# EUROPEAN PATENT APPLICATION

(11) **EP 4 596 037 A1**
(43) Date of publication of application: **06.08.2025**
(21) Application number: 23870348.2
(22) Date of filing: 14.09.2023
(51) Int. Cl.: A61N 5/10

(54) **NEUTRON CAPTURE THERAPY SYSTEM**

(30) Priority: 30.09.2022 CN 202211218032; 07.09.2023 CN 202311149807
(71) Applicant: Neuboron Therapy System Ltd., Xiamen, Fujian 361026 (CN)
(72) Inventor: LIU, Yuanhao, Nanjing, Jiangsu 211112 (CN); SHU, Diyun, Nanjing, Jiangsu 211112 (CN); LU, Weihua, Nanjing, Jiangsu 211112 (CN); GONG, Qiuping, Nanjing, Jiangsu 211112 (CN)
(74) Representative: Gunzelmann, Rainer
(86) International application number: PCT/CN2023/118681
(87) International publication number: WO 2024/067111

(57) **Abstract**

A neutron capture therapy system. The neutron capture therapy system comprises: a charged particle generation device (100) used for generating charged particles; an accelerator (110) used for accelerating the charged particles; a neutron generation device (200) comprising a particle transmission part (210) and a neutron generation part (220) provided in a first end part (211) of the particle transmission part (210), wherein the charged particles can pass through the particle transmission part (210) and interact with the neutron generation part (220) to generate neutrons; a beam shaping body (300) having an accommodating cavity, wherein the accommodating cavity comprises a first surface (S1) and a second surface (S2) intersecting with the first surface (S1), and the first end part (211) is provided in the accommodating cavity; and a first fitting part (230) provided between the first surface (S1) of the accommodating cavity and the outer surface of the particle transmission part (210), wherein the first fitting part (230) is configured to at least surround part of the particle transmission part (210). According to the neutron capture therapy system, the first fitting part (230) is provided between the first surface (S1) of the accommodating cavity and the outer surface of the particle transmission part (210), so that deflected neutrons are reflected to a preset direction, and the epithermal neutron flux for treatment is improved.

## Description

### TECHNICAL FIELD

The present disclosure relates to the technical field of radioactive ray irradiation, and in particular to a neutron capture therapy system.

### BACKGROUND

With the development of atomics, the radiotherapy such as the cobalt-60, the linear accelerator, and the electron beam has become one of major means to treat cancers. However, the conventional photon or electron therapy is restricted by physical conditions of radioactive rays. Specifically, while tumor cells are killed, a large number of normal tissues on a beam path are damaged. Due to different sensitivities of the tumor cells for the radioactive rays, the conventional radiotherapy is often undesirable to treat radioresistant malignant tumors (such as glioblastoma multiforme and melanoma).

In order to reduce radiation damages to the normal tissues surrounding the tumor, the target therapy in chemotherapy has been employed in the radiotherapy. For the highly radioresistant tumor cells, the radiotherapy with high relative biological effectiveness (RBE), including the proton therapy, the heavy particle therapy, and the neutron capture therapy, has also been developed actively. With specific aggregation of boron-containing drugs in tumor cells, and in cooperation with accurate neutron beam control, boron neutron capture therapy (BNCT) in neutron capture therapy serves as a better alternative to treat the cancers.

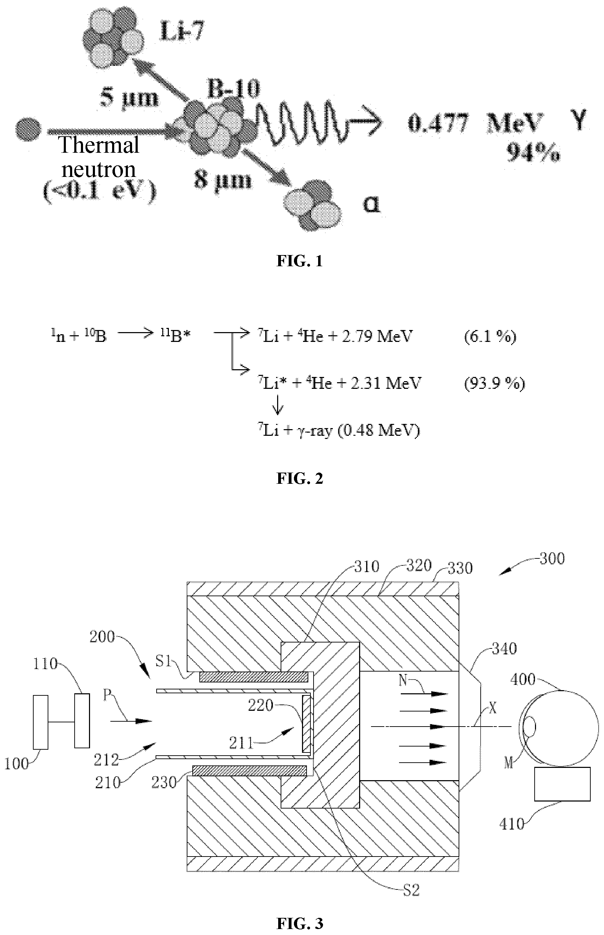

According to the BNCT, with a large capture cross section of the boron (¹⁰B)-containing drug for thermal neutrons, and through the ¹⁰B(n,α)⁷Li neutron capture reaction and the nuclear fission reaction, ⁴He and ⁷Li heavy charged particles are generated. FIG. 1 and FIG. 2 respectively illustrate a schematic view of a boron neutron capture reaction and an equation of a nuclear reaction in ¹⁰B(n,α)⁷Li neutron capture. The two heavy charged particles have an average energy of about 2.33 MeV, and have characteristics of the high linear energy transfer (LET), and the short range. The alpha particle has the LET of 150 keV/µm, and the range of 8 µm. The ⁷Li heavy charged particle has the LET of 175 keV/µm, and the range of 5 µm. The total range of the two particles is approximately equivalent to a cell size, such that the radiation damage to an organism can be limited to a cell level. When the boron-containing drug is aggregated to the tumor cells selectively, and an appropriate neutron source is provided, a purpose of locally killing the tumor cells on premise of no serious damage to normal tissues can be achieved.

According to the conventional art, a proton beam is accelerated to form accelerated charged particles. The accelerated charged particles are interacted with a target to form a neutron beam for treatment. However, the obtained neutron beam has an undesirable neutron flux or unduly high fast neutron contamination to cause the unsatisfactory treatment effect.

### SUMMARY

In view of technical problems of an undesirable neutron flux or unduly high fast neutron contamination of the neutron beam, the present disclosure provides a neutron capture therapy system.

A first aspect of the present disclosure provides a neutron capture therapy system, including a charged particle generation device configured to generate charged particles; an accelerator configured to accelerate the charged particles; a neutron generation device including a particle transmission portion and a neutron generation portion provided in a first end of the particle transmission portion, where the charged particles are capable of being interacted with the neutron generation portion through the particle transmission portion to generate neutrons; a beam shaping assembly (BSA) provided with an accommodating cavity, where the accommodating cavity includes a first surface and a second surface intersecting with the first surface, and the first end is provided in the accommodating cavity; and a first fitting portion provided between the first surface of the accommodating cavity and an outer surface of the particle transmission portion, where the first fitting portion surrounds at least a part of the particle transmission portion. By reflecting a deviated neutron back to a principal axis of a neutron beam, an amount of neutrons on the principal axis is increased. The BSA is configured to improve quality of the neutron beam, such that an epithermal neutron flux for treatment can be increased.

In an embodiment, at least a part of the first fitting portion is attached to the outer surface of the particle transmission portion. There is no gap between the first fitting portion and the particle transmission portion. This prevents a loss of the neutrons in the gap, enhances the reflection effect of the first fitting portion for the neutrons, and increases the epithermal neutron flux for the treatment.

In an embodiment, a first accommodating groove is formed in the first fitting portion; and at least a cooling tube or a cable is capable of passing through the first accommodating groove. When a cooling medium is provided in the cooling tube, the cooling medium can cool the neutron generation portion. This prevents the neutron generation portion from overheating when the charged particle beam is reacted with the neutron generation portion. Alternatively, when the particle transmission portion is to be provided with a sensor, a cable of the sensor can pass through the first accommodating groove to transmit a data signal to the outside

In an embodiment, the particle transmission portion is a hollow cylinder; and at least a part of the first fitting portion surrounds a cylindrical outer surface of the particle transmission portion. The hollow cylinder facilitates transmission of the charged particle beam. Compared with a cubic particle transmission portion, the cylindrical particle transmission portion can reduce collision of the charged particles against the sidewall, which increases a number of charged particles reaching the neutron generation portion.

In an embodiment, a material of the first fitting portion includes one or more of a group consisting of lead, graphite, and Teflon. The lead, the graphite, and the Teflon show a high elastic scattering cross section and a low absorption cross section in an energy range of epithermal neutrons, as well as a good reflectivity for the neutrons, such that the deviated neutron can be reflected to the principal axis of the neutron beam.

In an embodiment, the first fitting portion includes at least a first fitting body and a second fitting body; the first fitting body and the second fitting body are made of a same material or different materials; and the first fitting body and the second fitting body are capable of being connected to each other. The connection herein may be bonding, spliced connection, or other common connection manners known to those skilled in the art. To reflect the neutron to different positions behind the neutron generation portion, different energies are required, and corresponding to-be-selected optimal reflecting materials are also different.

In an embodiment, the neutron capture therapy system further includes a second fitting portion provided between the particle transmission portion and the second surface of the accommodating cavity. The second fitting portion is provided in front of the first end of the particle transmission portion, such that the neutrons generated by the neutron generation portion pass through the second fitting portion. By this time, the second fitting portion can adjust the quality of the neutron beam to optimize the treatment effect.

In an embodiment, at least a part of the first fitting portion is attached to the second fitting portion. The first fitting portion and the second fitting portion form a tight enclosed structure. This can reflect the neutron back to the principal axis of the neutron beam, and can improve the quality of the obtained neutron beam, thereby achieving the better treatment effect.

In an embodiment, the first fitting portion surrounds at least all of the particle transmission portion located in the accommodating cavity. This increases a reflective area of the first fitting portion, ensures that the neutron in the accommodating cavity can be reflected by the first fitting portion to the principal axis of the neutron beam, and effectively maximizes the epithermal neutron flux.

In an embodiment, the first fitting portion is provided on the particle transmission portion through a fastener. This can fix the first fitting portion, and can ensure that the first fitting portion and the particle transmission portion are attached without the gap.

A second aspect of the present disclosure provides a neutron capture therapy system, including a charged particle generation device configured to generate charged particles; an accelerator configured to accelerate the charged particles; a neutron generation device including a particle transmission portion and a neutron generation portion provided in a first end of the particle transmission portion, where the charged particles are capable of being interacted with the neutron generation portion through the particle transmission portion to generate neutrons; a BSA provided with an accommodating cavity, where the accommodating cavity includes a first surface and a second surface intersecting with the first surface, and the first end is provided in the accommodating cavity; and a second fitting portion provided between the second surface of the accommodating cavity and the particle transmission portion. The neutron beam generated by the neutron generation portion can pass through the second fitting portion. The second fitting portion adjusts quality of the neutron beam to improve the treatment effect.

In an embodiment, the second fitting portion is provided with a first end surface and a second end surface; the first end surface abuts against the second surface of the accommodating cavity; and at least a part of the second end surface abuts against the particle transmission portion. With the abutment, the second fitting portion is attached to the second surface of the accommodating cavity and the particle transmission portion at the same, without a gap. This reduces a loss of the neutrons, and improves the quality of the neutron beam.

In an embodiment, the second fitting portion has a same shape and a same size as the second surface of the accommodating cavity. The second fitting portion can completely fill the accommodating cavity, and is attached to the second surface of the accommodating cavity, such that the second surface is completely covered, and all neutrons can pass through the second fitting portion.

In an embodiment, the neutron capture therapy system further includes a first fitting portion; the first fitting portion is provided between the first surface of the accommodating cavity and an outer surface of the particle transmission portion; and the first fitting portion surrounds at least a part of the particle transmission portion. With the first fitting portion and the second fitting portion, a deviated neutron can be reflected back to a principal axis of the neutron beam to improve the quality of the neutron beam.

In an embodiment, at least a part of the first fitting portion is attached to the second fitting portion. The first fitting portion and the second fitting portion form a tight enclosed structure, thereby surrounding the first end of the particle transmission portion without a gap.

In an embodiment, a material of the second fitting portion includes one or more of a group consisting of magnesium fluoride, D₂O, AlF₃, Fluental, CaF₂, Li₂CO₃, MgF₂, Al₂O₃, and Al. The Fluental is a mixture formed by the Al, the MgF₂, and LiF at a preset portion. These materials can adjust energies of fast neutrons to form epithermal neutrons for treatment, thereby improving the quality of the neutron beam generated by the neutron generation portion.

In an embodiment, the second fitting portion includes a third fitting body and a fourth fitting body connected to the third fitting body.

In an embodiment, the BSA includes a moderator configured to slow the neutrons generated by the neutron generation portion down to an energy range of epithermal neutrons; the fourth fitting portion surrounds the third fitting body; and the third fitting body and the moderator are made of a same material. The neutron generation portion corresponds to the third fitting body. The fourth fitting body supports the third fitting body. The quality of the neutron beam generated by the neutron generation portion can be improved through the third fitting body.

According to the neutron capture therapy system, the first fitting portion is provided between the first surface of the accommodating cavity and the outer surface of the particle transmission portion, and at least a part of the first fitting portion surrounds the particle transmission portion, such that the deviated neutron is reflected back to the principal axis of the neutron beam, thereby increasing an amount of neutrons on the principal axis. The BSA is configured to improve the quality of the neutron beam, such that the epithermal neutron flux for treatment can be increased. The second fitting portion is provided between the second surface of the accommodating cavity and the end surface of the particle transmission portion. This improves the neutron slowing-down efficiency of the BSA, reduces the fast neutron contamination, and achieves better quality of the neutron beam.

### BRIEF DESCRIPTION OF THE DRAWINGS

FIG. 1 is a schematic view of a boron neutron capture reaction;
FIG. 2 illustrates an equation of a nuclear reaction in ¹⁰B(n,α)⁷Li neutron capture;
FIG. 3 is a schematic structural view of a neutron capture therapy system according to an embodiment;
FIG. 4 is a schematic structural view illustrating that a first fitting portion is attached to a particle transmission portion according to an embodiment;
FIG. 5 is a schematic structural view illustrating that a first fitting portion is provided with a first accommodating groove according to an embodiment;
FIG. 6 is a schematic structural view illustrating that a first fitting portion includes a first fitting body and a second fitting body according to an embodiment;
FIG. 7 is a schematic structural view illustrating that a first fitting portion includes four fitting bodies according to an embodiment;
FIG. 8 is a schematic structural view illustrating that a first fitting portion is provided with a first accommodating groove according to another embodiment;
FIG. 9 is a schematic structural view when there is a gap between a first fitting portion and a particle transmission portion according to an embodiment;
FIG. 10 is a schematic structural view when a first fitting portion and a second fitting portion are provided at the same time according to an embodiment;
FIG. 11 is a schematic structural view when a first fitting portion is attached to the second fitting portion according to an embodiment;
FIG. 12 is a schematic structural view of a neutron capture therapy system according to another embodiment;
FIG. 13 is a schematic structural view when a second fitting portion is provided according to an embodiment;
FIG. 14 is a schematic structural view when a cooling tube is special-shaped according to an embodiment;
FIG. 15 is a schematic structural view illustrating that a second fitting portion includes a third fitting body and a fourth fitting body according to an embodiment; and
FIG. 16 is a schematic structural view of a second fitting portion according to an embodiment.

In the figures:
100: charged particle generation device, 110: accelerator, 200: neutron generation device, 210: particle transmission portion, 220: neutron generation portion, 230: first fitting portion, 231: first fitting body, 232: second fitting body, 233: first accommodating groove, 240: cooling tube, 250: second fitting portion, 251: third fitting body, 252: fourth fitting body, 253: second accommodating groove, 254: tube fastener, 300: BSA, 310: moderator, 320: reflector, 330: radiation shield, 340: collimator, 400: irradiated body, 410: treatment table, S1: first surface, S2: second surface, P: charged particle beam, N: neutron beam, M: irradiation position, and X: principal axis.

### DETAILED DESCRIPTION OF THE EMBODIMENTS

To make the above objectives, features and advantages of the present disclosure more comprehensible, the specific implementations of the present disclosure are described in detail below with reference to the drawings. Many details are provided in the following description in order for a thorough understanding of the present disclosure. However, the present disclosure may be implemented in many other ways other than those described herein, and those skilled in the art may make similar improvements without departing from the connotation of the present disclosure. Therefore, the present disclosure is not limited to the specific embodiments disclosed below.

A mixed radiation field is generated by a neutron source in BNCT, that is, the generated beam includes neutrons, photons and other radioactive rays having energies from low to high. As for the BNCT on deep-seated tumors, except the epithermal neutrons, the more the remaining radioactive rays, the greater the proportion causing non-selective dose deposition in the normal tissues, namely damaging the normal tissues. Therefore, in addition to the epithermal neutrons for treatment, the radioactive rays causing the unnecessary dose should be reduced as much as possible.

For neutron sources in the clinical BNCT, the International Atomic Energy Agency (IAEA) provides five suggestions for beam quality factors in air. The five suggestions can be used to compare advantages and disadvantages of different neutron sources, and used as a reference basis to select a neutron generation method and design a BSA. The five suggestions are as follows:
Epithermal neutron flux > 1*10⁹ n/cm²s;
Fast neutron contamination < 2*10⁻¹³ Gy-cm²/n;
Photon contamination<2*10⁻¹³ Gy-cm²/n;
Thermal to epithermal neutron flux ratio < 0.05; and
Epithermal neutron current to flux ratio > 0.7

Note: The epithermal neutrons have an energy range between 0.5 eV to 10 keV, the thermal neutrons have an energy range of less than 0.5 eV, and the fast neutrons have an energy range of greater than 10 keV. Certainly, the present disclosure is not limited to only use this standard as the reference basis, and may also use other standards known to those skilled in the art as the reference basis.

### 1. Epithermal neutron flux:

The neutron flux and the concentration of the boron-containing drug in the tumor determine the clinical treatment time. If the concentration of the boron-containing drug in the tumor is high enough, lower requirements may be imposed on the neutron flux. Conversely, if the concentration of the boron-containing drug in the tumor is low, high-flux epithermal neutrons are required to deliver an enough dose to the tumor. According to the IAEA, the epithermal neutron flux is greater than 1*10⁹ n/cm²s. For the existing boron-containing drug, the treatment time of the neutron beam may be controlled within one hour approximately. With the short treatment time, besides the advantages in patient positioning and comfort level, limited detention time of the boron-containing drug in the tumor may also be effectively used.

### 2. Fast neutron contamination:

The unnecessary dose of the fast neutrons to the normal tissues is considered as contamination. This dose is positively correlated with the neutron energy. Thus, the fast neutrons in the neutron beam should be reduced as much as possible. The fast neutron contamination is defined as a fast-neutron dose accompanied in the unit epithermal neutron flux. According to the IAEA, the fast neutron contamination should be less than 2*10⁻¹³ Gy-cm²/n.

### 3. Photon contamination (gamma-ray contamination):

With strongly penetrating radiation, gamma-rays will cause dose deposition non-selectively to all tissues on the beam path. To design the neutron beam, it is essential to reduce the gamma-rays. The gamma-ray contamination is defined as a gamma-ray dose in the unit epithermal neutron flux. According to the IAEA, the gamma-ray contamination should be less than 2*10⁻¹³Gy-cm²/n.

### 4. Thermal to epithermal neutron flux ratio:

Due to fast decay and poor penetrability of the thermal neutrons, energies of the thermal neutrons in the body are largely deposited on skin tissues. Except that epidermal tumors such as melanoma are required to use the thermal neutrons as a neutron source in the BNCT, the thermal neutrons should be reduced for deep-seated tumors such as brain tumor. According to the IAEA, the thermal to epithermal neutron flux ratio should be less than 0.05.

### 5. Epithermal neutron current to flux ratio:

The epithermal neutron current to flux ratio indicates directivity of the beam. The greater the epithermal neutron current to flux ratio, the better the forward directivity of the neutron beam. The neutron beam with the strong forward directivity can reduce a dose to the surrounding normal tissues due to neutron diffusion, and further improve the treatable depth and the positioning flexibility. According to the IAEA, the epithermal neutron current to flux ratio should be greater than 0.7.

Referring to FIG. 3, FIG. 3 is a schematic structural view of a neutron capture therapy system according to an embodiment. The neutron capture therapy system includes a charged particle generation device 100, an accelerator 110, a neutron generation device 200, and a BSA 300. The charged particle generation device 100 is configured to generate charged particles, such as protons and deuterons. The accelerator 110 is configured to accelerate the charged particles (such as the protons and the deuterons) to generate a charged particle beam P such as a proton line. The charged particle beam P is interacted with the neutron generation device 200 to generate neutrons, thereby forming a neutron beam N. The BSA 300 is configured to adjust quality of the neutron beam N. FIG. 3 further illustrates a positional relationship when the neutron beam N is used to irradiate an irradiated body 400 in some embodiments. The irradiated body 400 is located in an exiting direction of the neutron beam N. The irradiated body 400 may be a human or an animal. An irradiation position M may be a position where tumor cells are located. The irradiated body 400 may be provided on a treatment table 410. The treatment table 410 may be a treatment couch, a treatment chair or other types of supporting devices for supporting the irradiated body 400.

The neutron generation device 200 may include a particle transmission portion 210, a neutron generation portion 220, and a first fitting portion 230 surrounding at least a part of the particle transmission portion 210. The particle transmission portion 210 is a hollow tubular structure, and includes a first end 211 and a second end 212. As is known to those skilled in the art, the particle transmission portion 210 may be a square or a cylinder, provided that the charged particle beam P can pass through the particle transmission portion. The neutron generation portion 220 may be accommodated in the first end 211 of the particle transmission portion 210. Entering from the second end 212 of the particle transmission portion 210, the charged particle beam P may reach the neutron generation portion 220 in the first end 211 through the particle transmission portion 210, and is interacted with the neutron generation portion 220 to generate the neutrons to form the neutron beam N. The neutron beam N defines a principal axis X. In the figure and the following description, the direction of the neutron beam N does not represent an actual moving direction of the neutrons, but an overall movement trend of the neutron beam N. The first fitting portion 230 is configured to reflect a neutron deviated from the principal axis X back to the principal axis X, thereby increasing an epithermal neutron flux.

The neutron generation portion 220 may be a target. After passing through the particle transmission portion 210, an accelerated charged particle beam P takes place a nuclear reaction with the target to generate the neutrons. The ideal target features a high neutron yield, a capability of generating neutrons with energies close to the energy range of epithermal neutrons, no excessive long-range radiation, safety, a cheap cost, an easy operation, a high temperature resistance, etc. However, as a matter of fact, a nuclear reaction meeting all requirements cannot be found. In some embodiments of the present disclosure, a lithium-containing target and a ⁷Li(p,n)⁷Be nuclear reaction are used. However, as is known to those skilled in the art, the target T may also be made of a metal material other than Li and Be, such as Ta or W and an alloy thereof. The accelerator 110 may be a linear accelerator, a cyclotron, a synchrotron or an electrostatic accelerator.

The first fitting portion 230 surrounds at least a part of the particle transmission portion 210. The first fitting portion 230 may be attached to the particle transmission portion 210, with a gap from a first surface S1 of an accommodating cavity. The first fitting portion may be attached to the first surface S1 of the accommodating cavity, with a gap from the particle transmission portion 210. The first fitting portion may further completely fill a gap between the particle transmission portion 210 and the first surface S1 of the accommodating cavity. The first fitting portion 230 may be provided along an axial direction of the particle transmission portion 210. The first fitting portion 230 may match with the particle transmission portion 210 in shape and size. In an embodiment, the particle transmission portion 210 is a cylinder. The first fitting portion 230 may also be a cylinder, with a size capable of surrounding the particle transmission portion 210 by one circumference. The first fitting portion partially covers the particle transmission portion 210 in the axial direction of the particle transmission portion 210. The first fitting body 230 may be made of a material with a high elastic scattering cross section and a low absorption cross section in an energy range of the epithermal neutrons, such as one or more of a group consisting of lead, graphite, and Teflon.

The BSA 300 is provided with the hollow accommodating cavity. The accommodating cavity may be a square or a cylinder, and may match with the neutron generation device 200 in shape. In an embodiment, the neutron generation device 200 is a cylinder. Correspondingly, the accommodating cavity is also a cylinder, and includes the first surface S1 serving as a side of the cylinder and the second surface S2 intersecting with the first surface S1 and serving as a bottom of the cylinder. The first end 211 of the neutron generation device 200 provided with the neutron generation portion 220 extends into the accommodating cavity. A side of the particle transmission portion 210 corresponds to the first surface S1. An end surface of the particle transmission portion 210 corresponds to the second surface S2. When the first fitting portion 230 is provided at the side of the particle transmission portion 210, the first fitting portion 230 is located between the side of the particle transmission portion 210 and the first surface S1, thereby filling a gap between the side of the particle transmission portion 210 and the first surface S1 of the accommodating cavity. The first fitting portion 230 may be close to the first surface S1, with a gap from the particle transmission portion 210, or completely fill the gap between the particle transmission portion 210 and the first surface S1. In this way, the charged particle line P is irradiated onto the neutron generation portion 220 to generate the neutrons. With the quality improved through the BSA 300, the neutrons form the neutron beam N.

Referring also to FIG. 3, the BSA 300 may include a moderator 310, a reflector 320, and a radiation shield 330. The neutrons generated by the neutron generation device 200 have a very wide energy spectrum. Except that the epithermal neutrons meet the treatment requirement, neutrons and photons of other types are to be reduced as much as possible, so as not to hurt the operator or normal tissues of the patient. Hence, concerning the neutrons from the neutron generation device 200, energies of fast neutrons are adjusted by the moderator 310 to the energy range of the epithermal neutrons. The moderator 310 is made of a material having a large action cross section with the fast neutrons but a small action cross section with the epithermal neutrons, such as at least one of D₂O, Al, AlF₃, MgF₂, CaF₂, LiF, Li₂CO₃ or Al₂O₃. The reflector 320 surrounds the moderator 310, and is configured to reflect a neutron diffused through the moderator 310 back to the principal axis X of the neutron beam N to improve a utilization rate of the neutron. The reflector 320 is made of a material with a strong neutron reflectivity, such as at least one of Pb or Ni. The reflector 320 and the moderator 310 collectively define the hollow accommodating cavity, which is configured to accommodate the particle transmission portion 210 with the end provided with the neutron generation portion 220. At least partially surrounding the reflector 320, the radiation shield 330 is configured to shield a leaked neutron and a leaked photon to reduce a dose to a normal tissue in a non-irradiated region. A material of the radiation shield 330 includes at least one of a photon shielding material and a neutron shielding material, such as the photon shielding material (Pb) and the neutron shielding material (PE). A collimator 340 may further be provided. Provided at a beam outlet of the BSA 300, the collimator 340 may be configured to gather the neutron beam N, such that the neutron beam N has a high targeting ability in the treatment.

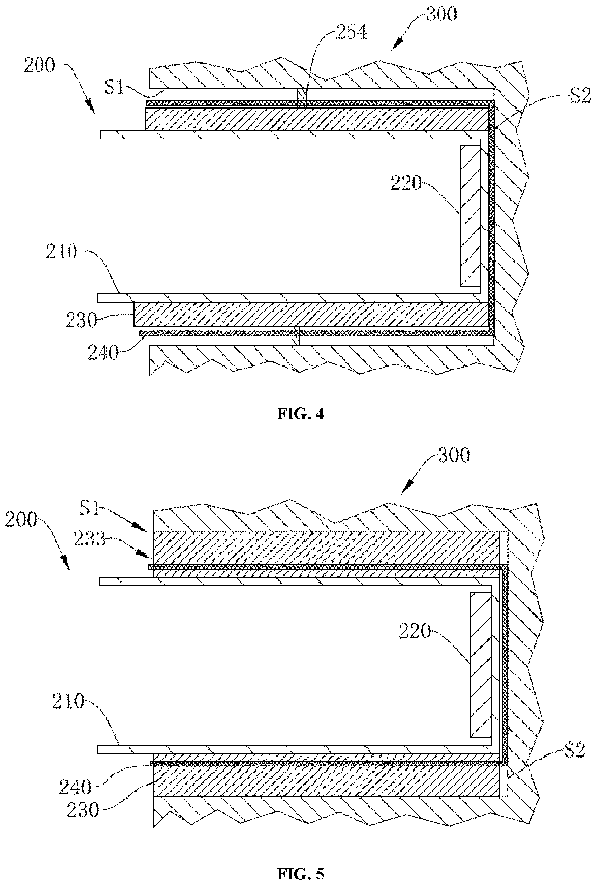

Referring to FIG. 4, FIG. 4 is a schematic structural view illustrating that the first fitting portion 230 is attached to the particle transmission portion 210 according to an embodiment. In some embodiments, the first fitting portion 230 may be partially or totally attached to an outer surface of the particle transmission portion 210. This can reflect neutrons generated by interaction between the charged particle beam P and the neutron generation portion 220 back to the principal axis X, thereby preventing the neutrons from escaping from the side of the particle transmission portion 210, and increasing the epithermal neutron flux for the treatment. The first fitting portion 230 may be provided on the particle transmission portion 210 through a fastener. The fastener may be a locking workpiece surrounding the first fitting portion 230, such as a binding band and a hoop. Through the fastener, the first fitting portion 230 may be attached to the particle transmission portion 210.

When a nuclear reaction occurs between the charged particle beam P and the neutron generation portion 220 to generate the neutrons, a large amount of heat is produced. Hence, the first end 211 of the particle transmission portion 210 where the neutron generation portion 220 is located is to be cooled in a manner known to those skilled in the art, such as gas cooling or water cooling. A cooling medium is transmitted to the first end 211 of the particle transmission portion 210 through a cooling tube 240. The cooling medium flows through a part of the cooling tube 240 close to the neutron generation portion 220 to absorb the heat produced by the nuclear reaction, and flows out from the other end of the particle transmission portion, thereby realizing cooling. When the cooling tube 240 is provided, the cooling tube 240 may extend into the accommodating cavity through the gap between the first fitting portion 230 and the first surface S1. The cooling tube 240 may be located between the first end 211 of the particle transmission portion 210 and the second surface S2, and is preferably attached to the second surface S2. The cooling tube may be fastened by a tube fastener 254.

Certainly, in other optional embodiments, the cooling tube 240 may also be assembled on the particle transmission portion 210. A part of the cooling tube 240 is close to the neutron generation portion 220 and provided outside the first end 211 of the particle transmission portion 210, with a shape matching with a cross section of the first end 211. This facilitates uniform and full heat exchange between the cooling medium and the neutron generation portion 220. The cooling tube 240 may be assembled on the particle transmission portion 210 in a manner known to those skilled in the art, such as fastening, welding, and bonding. The first fitting portion 230 matches with a periphery of the particle transmission portion 210 in shape, and specifically includes an accommodating groove for accommodating the cooling tube 240. The first fitting portion 230 may be partially or totally attached to the outer surface of the particle transmission portion 210. This can reflect neutrons generated by interaction between the charged particle beam P and the neutron generation portion 220 back to the principal axis X, thereby preventing the neutrons from escaping from the side of the particle transmission portion 210, and increasing the epithermal neutron flux for the treatment. Preferably, at least a part of the first fitting portion 230 is attached to the outer surface of the particle transmission portion 210, and attached to the first surface S1 as much as possible when an assembly condition is met.

Referring also to FIG. 4, in an embodiment, the first fitting portion 230 surrounds at least all of the particle transmission portion 210 located in the accommodating cavity. By this time, a length of the first fitting portion 230 is greater than or equal to a depth of the accommodating cavity. This can improve reflection efficiency for the charged particle beam P. By surrounding all of the particle transmission portion 210, the charged particle beam P can be prevented from escaping from an incident direction to cause less neutrons to take place the nuclear reaction with the neutron generation portion, thereby increasing the final neutron flux.

Referring to FIG. 5, FIG. 5 is a schematic view illustrating that the first fitting portion 230 is provided with a first accommodating groove 233. When the first fitting portion 230 fills a part of the gap between the first surface S1 and the particle transmission portion 210, the gap not provided with the fitting portion may be provided with the cooling tube 240 (as shown in FIG. 4). In order to improve the reflection effect for the neutrons, the first fitting portion 230 may fill all of the gap between the first surface S1 and the particle transmission portion 210. By this time, the first fitting portion 230 may be provided therein with the first accommodating groove 233, which is configured to pass through the cooling tube 240 or the cable at the side of the particle transmission portion or other components protruding from the side of the particle transmission portion (not shown in the figure). The first accommodating groove may be a square groove, a circular groove or other special-shaped grooves with higher adaptability.

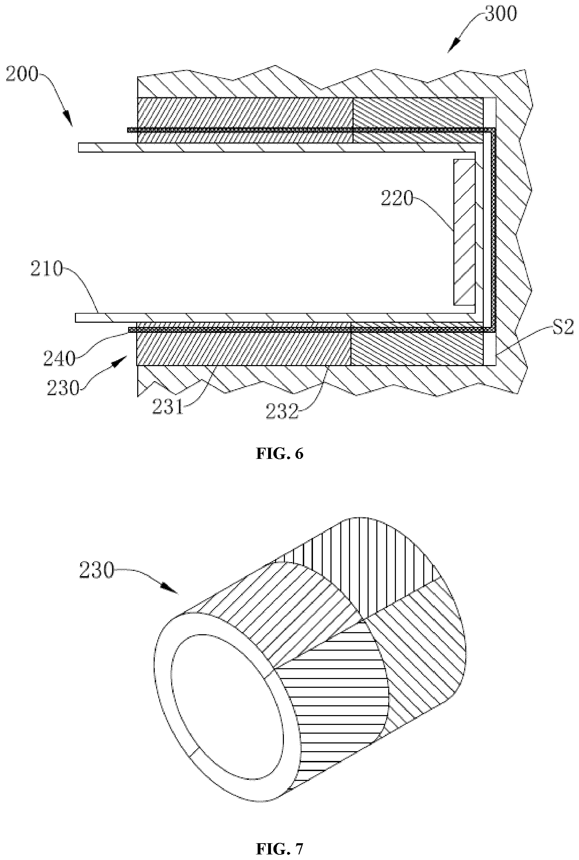

Referring to FIG. 6, FIG. 6 is a schematic structural view illustrating that the first fitting portion 230 includes a first fitting body 231 and a second fitting body 232 according to an embodiment. The first fitting body 231 and the second fitting body 232 may be made of a same material or different materials. For example, the first fitting body 231 and the second fitting body 232 may be made of a material with a strong neutron reflectivity such as lead, graphite, and Teflon.

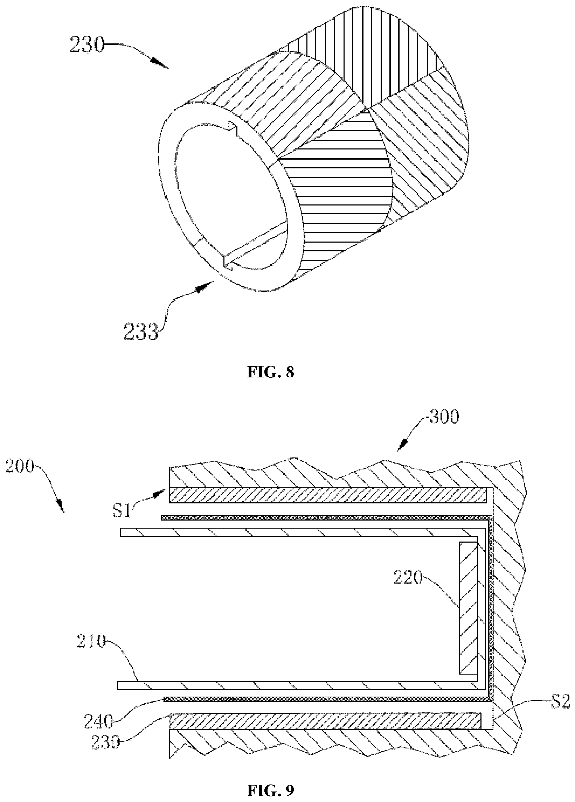

As is known to those skilled in the art, the first fitting portion 230 may not be limited to two fitting bodies, and may further include more fitting bodies. For example, FIG. 7 is a structural view further illustrating that the first fitting portion 230 includes four fitting bodies according to an embodiment. The four fitting bodies are connected to each other to form the whole first fitting portion 230. The four fitting bodies may be connected in a common manner known to those skilled in the art, such as bonding, splicing, fastening, and welding. The first fitting portion 230 may be fixed on the neutron generation device through a locking member, and may also be fixed on the neutron generation device by bonding, welding or other common assembly manners. FIG. 8 is a schematic structural view illustrating that the first accommodating groove 233 is formed in the first fitting portion 230 according to an embodiment. When the first fitting portion 230 surrounds the particle transmission portion 210, the cooling tube 240 may be provided in the first accommodating groove 233, thereby extending to the first end 211 of the particle transmission portion 210 in the accommodating groove to cool the neutron generation portion 220.

Monte Carlo N-particle (MCNP) software (a general-purposed software package developed by the LosAlamos National Laboratory based on the Monte Carlo method and used for calculating a transport problem of the neutron, photon, charged particle or coupled neutron/photon/charged particle in the three-dimensional (3D) complex geometric structure) is used to simulate and calculate a structure of the first fitting portion 230 surrounding the particle transmission portion 210 in different embodiments. Table 1 below shows influences of beam quality factors in air in different embodiments (the unit of each noun in the table is the same as the above, and will not be repeated herein).

In the first group, the first fitting portion 230 is not provided between the particle transmission portion 210 and the first surface S1. In the second group, the first fitting portion 230 is provided between the particle transmission portion 210 and the first surface S1. However, the first fitting portion 230 does not completely fill the gap between the particle transmission portion 210 and the first surface S1, as shown in FIG. 9. The first fitting portion 230 is close to the first surface S1, with a gap from the particle transmission portion 210. In the third group, the first fitting portion 230 is provided between the particle transmission portion 210 and the first surface S1. The first fitting portion 230 completely fills the gap between the particle transmission portion 210 and the first surface S1, as shown in FIG. 5. The first fitting portion 230 is made of the Teflon. In the fourth group, the first fitting portion 230 is provided between the particle transmission portion 210 and the first surface S1. The first fitting portion 230 completely fills the gap between the particle transmission portion 210 and the first surface S1. The first fitting portion 230 includes the first fitting body 231 and the second fitting body 232, as shown in FIG. 6. The first fitting body 231 is made of the Teflon, while the second fitting body 232 is made of the graphite. In the second group, the third group, and the fourth group, the first fitting portion 230 extends into the accommodating cavity by a same depth.

**Table 1: Beam quality in air**

| | First group | Second group | Third group | Fourth group |
|---|---|---|---|---|
| Epithermal neutron flux | 6.05E+08 | 6.74E+08 | 7.53E+08 | 6.85E+08 |
| Fast neutron contamination | 9.66E-13 | 8.65E-13 | 7.04E-13 | 8.28E-13 |

As can be seen from the above simulation and calculation results, by providing the first fitting portion 230 between the particle transmission portion 210 and the first surface S1, the epithermal neutron flux is increased significantly, and the fast neutron contamination is reduced significantly. Compared with the second group in which the first fitting portion 230 does not completely fill the gap between the particle transmission portion 210 and the first surface S1, the epithermal neutron flux is further increased in the third group in which the first fitting portion 230 completely fills the gap between the particle transmission portion and the first surface. For the fourth group in which the first fitting portion 230 is combined by the Teflon and the graphite, compared with the third group in which the Teflon serves as the whole first fitting portion 230, the epithermal neutron flux is decreased. However, compared with the case in which the first fitting portion 230 is not provided, the epithermal neutron flux is increased significantly. This indicates that the first fitting portion 230 may be made of a single material or a plurality of materials at the same time.

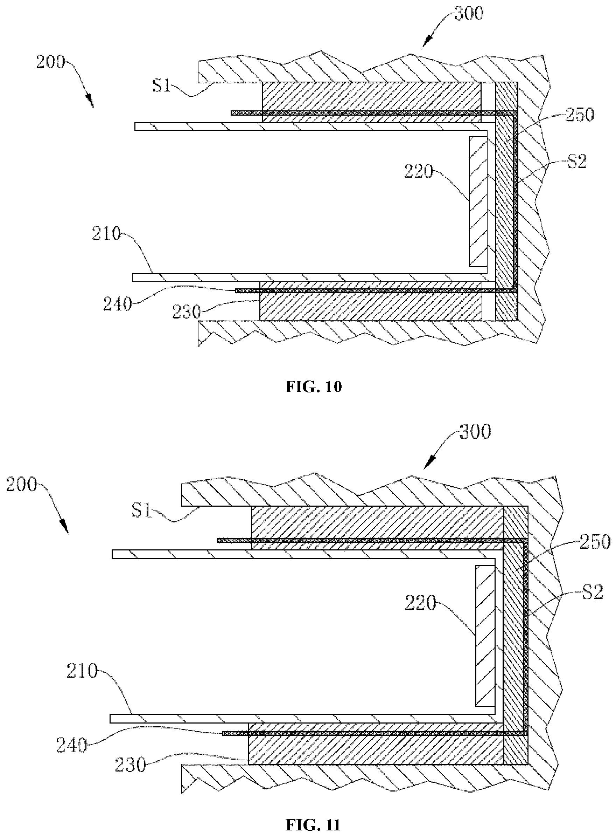

Referring to FIG. 10, FIG. 10 is a schematic structural view when a second fitting portion 250 is provided according to an embodiment. The second fitting portion 250 is located between the particle transmission portion 210 and the second surface S2 of the accommodating cavity. A plane of the second fitting portion 250 may be perpendicular or approximately perpendicular to the principal axis X. The second fitting portion may be provided along a radial direction of the particle transmission portion 210, such that the neutron beam N can pass through the second fitting portion 250. The second fitting portion 250 is configured to adjust the quality of the neutron beam N. A shape and a size of the second fitting portion 250 may depend on a bottom area of the particle transmission portion 210. The second fitting portion may have the same shape as the particle transmission portion 210, with the size greater than or equal to a size of a bottom of the particle transmission portion 210. In some embodiments, the shape and the size of the second fitting portion 250 may be the same or approximately the same as those of the second surface S2. For example, when the accommodating cavity is a cylinder, and the second surface S2 is a circle, the bottom of the second fitting portion 250 may be a circle having the same shape and same size as the second surface S2. By this time, the second fitting portion 250 is a cylinder. Before the neutron generation device 200 extends into the accommodating cavity, the second fitting portion 250 may be provided in the accommodating cavity and attached to the second surface S2. In other optional implementations, the second fitting portion 250 may also be fixed on the second surface S2 by bonding. By providing the second fitting portion 250 between the particle transmission portion 210 and the second surface S2 of the accommodating cavity, the fast neutron contamination can be reduced, and the maximal tumor dose and the treatment depth are increased. This improves the overall quality of the neutron beam to achieve the better treatment effect.

In the embodiment shown in FIG. 10, the first fitting portion 230 does not completely cover the particle transmission portion 210, and the first fitting portion 230 does not abut against the second fitting portion 250. That is, there is a gap between the first fitting portion 230 and the second fitting portion 250. This can also increase the neutron flux, and reduce the fast neutron contamination. Certainly, in other optional implementations, when the first fitting portion 230 does not completely cover the particle transmission portion 210, the first fitting portion 230 may abut against the second fitting portion 250, or the first fitting portion 230 may be close to the other end opposite to the first end of the accommodating cavity.

Referring to FIG. 11, FIG. 11 is a schematic structural view when the first fitting portion 230 is attached to the second fitting portion 250 according to an embodiment. The first fitting portion 230 is an annular cylindrical structure with a side surrounding at least a part of the particle transmission portion 210. An end surface of the first fitting portion 230 may be attached to the second fitting portion 250. The first fitting portion 230 and the second fitting portion 250 define an enclosed space. This improves the reflection effect for the neutrons, and reduces the fast neutron contamination.

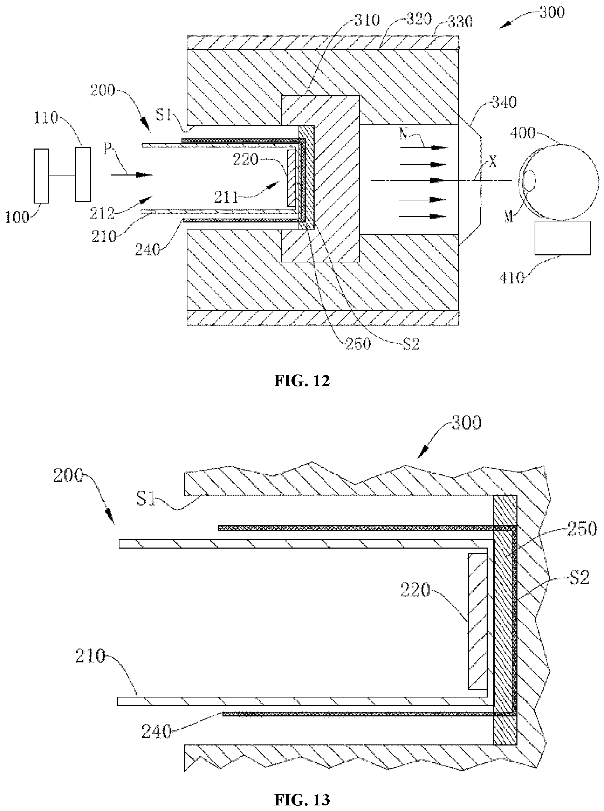

Referring to FIG. 12, FIG. 12 is a schematic structural view of a neutron capture therapy system according to an embodiment. The neutron capture therapy system includes a charged particle generation device 100, an accelerator 110, a neutron generation device 200, and a BSA 300. The charged particle generation device 100 is configured to generate charged particles, such as protons and deuterons. The accelerator 110 is configured to accelerate the charged particles (such as the protons and the deuterons) to generate a charged particle beam P such as a proton line. The charged particle beam P is interacted with the neutron generation device 200 to generate neutrons, thereby forming a neutron beam N. The BSA 300 is configured to adjust quality of the neutron beam N. FIG. 12 further illustrates a positional relationship when the neutron beam N is used to irradiate an irradiated body 400 in some embodiments. The irradiated body 400 is located in an exiting direction of the neutron beam N. The irradiated body 400 may be a human or an animal. An irradiation position M may be a position where tumor cells are located. The irradiated body 400 may be provided on a treatment table 410. The treatment table 410 may be a treatment couch, a treatment chair or other types of supporting devices for supporting the irradiated body 400. A cooling tube 240 may be fixed by a tube fastener 254 (not shown in the figure). The tube fastener 254 may abut against a second surface S2 of an accommodating cavity.

The neutron generation device 200 may include a particle transmission portion 210, a neutron generation portion 220, and a second fitting portion 250 configured to improve the beam quality. The particle transmission portion 210 is a hollow tubular structure, and includes a first end 211 and a second end 212. As is known to those skilled in the art, the particle transmission portion 210 may be a square or a cylinder, provided that the charged particle beam P can pass through the particle transmission portion. The neutron generation portion 220 may be accommodated in the first end 211 of the particle transmission portion 210. Entering from the second end 212 of the particle transmission portion 210, the charged particle beam P may reach the neutron generation portion 220 in the first end 211 through the particle transmission portion 210, and is interacted with the neutron generation portion 220 to generate the neutrons to form the neutron beam N. The neutron beam N defines a principal axis X. In the figure and the following description, the direction of the neutron beam N does not represent an actual moving direction of the neutrons, but an overall movement trend of the neutron beam N. The second fitting portion 250 may be provided outside the first end 211 of the particle transmission portion 210, and configured to adjust the quality of the neutron beam N.

The neutron generation portion 220 may be a target. After passing through the particle transmission portion 210, an accelerated charged particle beam P takes place a nuclear reaction with the target to generate the neutrons. The ideal target features a high neutron yield, a capability of generating neutrons with energies close to the energy range of epithermal neutrons, no excessive long-range radiation, safety, a cheap cost, an easy operation, a high temperature resistance, etc. However, as a matter of fact, a nuclear reaction meeting all requirements cannot be found. In some embodiments of the present disclosure, a lithium-containing target and a ⁷Li(p,n)⁷Be nuclear reaction are used. However, as is known to those skilled in the art, the target T may also be made of a metal material other than Li and Be, such as Ta or W and an alloy thereof. The accelerator 110 may be a linear accelerator, a cyclotron, a synchrotron or a synchrocyclotron.

The BSA 300 is provided with the hollow accommodating cavity. The accommodating cavity may be a square or a cylinder, and may match with the neutron generation device 200 in shape. In an embodiment, the neutron generation device 200 is a cylinder. Correspondingly, the accommodating cavity is also a cylinder, and includes a first surface S1 serving as a side of the cylinder and the second surface S2 intersecting with the first surface S1 and serving as a bottom of the cylinder. The first end 211 of the neutron generation device 200 provided with the neutron generation portion 220 extends into the accommodating cavity. A side of the particle transmission portion 210 corresponds to the first surface S1 of the accommodating cavity. A bottom of the particle transmission portion 210 located at one side of the first end 211 corresponds to the second surface S2 of the accommodating cavity. The second fitting portion 250 is located between the bottom of the particle transmission portion 210 and the second surface S2 of the accommodating cavity, thereby filling a gap between the side of the particle transmission portion 210 and the second surface S2 of the accommodating cavity. A material of the second fitting portion 250 may include one or more of a group consisting of magnesium fluoride, D₂O, AlF₃, Fluental, CaF₂, Li₂CO₃, MgF₂, Al₂O₃, and Al. The Fluental is a composite prepared by a mixture z formed by the Al, the AlF₃, and LiF at a preset proportion. The neutrons generated by the neutron generation portion 220 enter the second fitting portion 250 first and then enter the BSA 300 to adjust the beam quality. The neutrons generated by the neutron generation portion 220 are adjusted twice by the second fitting portion 250 and the BSA 300.

Referring also to FIG. 12, the BSA 300 may include a moderator 310, a reflector 320, and a radiation shield 330. The neutrons generated by the neutron generation device 200 have a very wide energy spectrum. Except that epithermal neutrons meet the treatment requirement, neutrons and photons of other types are to be reduced as much as possible, so as not to hurt the operator or normal tissues of the patient. Hence, concerning the neutrons from the neutron generation device 200, energies of fast neutrons are adjusted by the moderator 310 to the energy range of the epithermal neutrons. The moderator 310 is made of a material having a large action cross section with the fast neutrons but a small action cross section with the epithermal neutrons, such as at least one of D₂O, Al, AlF₃, MgF₂, CaF₂, LiF, Li₂CO₃ or Al₂O3. The reflector 320 surrounds the moderator 310, and is configured to reflect a neutron diffused through the moderator 310 back to the principal axis X of the neutron beam N to improve a utilization rate of the neutron. The reflector 320 is made of a material with a strong neutron reflectivity, such as at least one of Pb or Ni. The reflector 320 and the moderator 310 collectively define the hollow accommodating cavity, which is configured to accommodate the particle transmission portion 210 with the end provided with the neutron generation portion 220. At least partially surrounding the reflector 320, the radiation shield 330 is configured to shield a leaked neutron and a leaked photon to reduce a dose to a normal tissue in a non-irradiated region. A material of the radiation shield 330 includes at least one of a photon shielding material and a neutron shielding material, such as the photon shielding material (Pb) and the neutron shielding material (PE). A collimator 340 may further be provided. Provided at a beam outlet of the BSA 300, the collimator 340 may be configured to gather the neutron beam N, such that the neutron beam N has a high targeting ability in the treatment.

Referring to FIG. 13, FIG. 13 is a schematic structural view of the second fitting portion 250 according to an embodiment. The second fitting portion 250 includes a first end surface and a second end surface. The first end surface of the second fitting portion 250 abuts against the second surface S2 of the accommodating cavity. The second end surface of the second fitting portion 250 abuts against the particle transmission portion 210. The second fitting portion 250 is tightly attached to the particle transmission portion 210 and the second surface S2 of the accommodating cavity to completely fill the gap, thereby reducing the loss of the neutrons. A second accommodating groove 253 configured to allow the cooling tube 240, a cable and the like to pass through may be formed in the second fitting portion 250. The cooling tube 240 can pass through the second fitting portion 250, and cool the neutron generation portion 220 in the particle transmission portion 210. The second accommodating groove 253 may be a square groove, a circular groove or other special-shaped grooves with higher adaptability.

Referring also to FIG. 13, in some embodiments, the second fitting portion 250 has a same shape and a same size as the second surface S2 of the accommodating cavity, such that the second fitting portion 250 is completely attached to the second surface S2 of the accommodating cavity. For example, when the accommodating cavity is a cylinder, and the second surface S2 is a circle, the second fitting portion 250 is a circle having a bottom same as the second surface S2 of the accommodating cavity in size. The second fitting portion 250 is a cylinder attached to and completely covering the second surface S2 of the accommodating cavity.

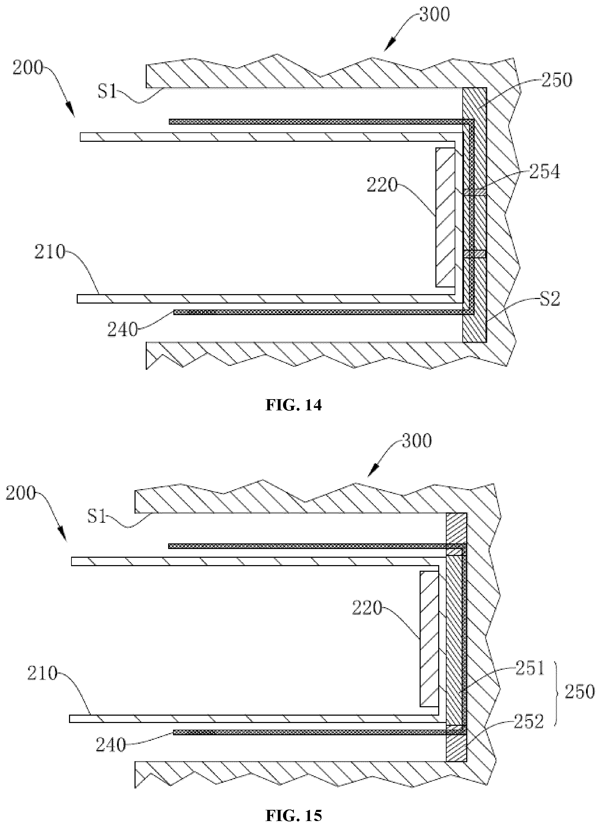

Referring to FIG. 14, FIG. 14 is a schematic structural view when the second accommodating groove 253 is a special-shaped groove according to an embodiment. As shown, a fixture configured to fix the cooling tube is provided on an end surface of the neutron generation device 200, such that the end surface of the neutron generation device 200 becomes rough. The second accommodating groove 253 is configured to accommodate the cooling tube 240 and the fixture, thereby filling the gap between the neutron generation device 200 and the second surface S2.

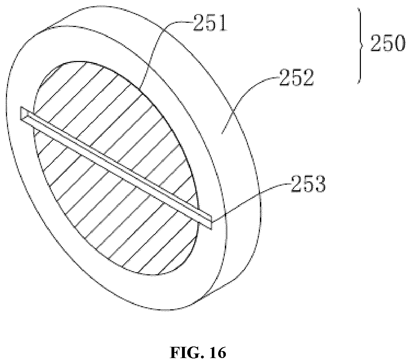

In other optional implementations, in order to ensure cooling efficiency, the cooling tube 240 is a special-shaped structure of an annular shape, a circular shape or a wavy shape. In order to match with the shape of the cooling tube 240, the second accommodating groove 253 may be a special-shaped groove matching with the cooling tube 240 in shape and size. When the first transmission portion 210 extends into the accommodating groove, the cooling tube 240 can match with the second accommodating groove 253. Referring to FIG. 15, FIG. 15 illustrates that the second fitting portion 250 includes a third fitting body 251 and a fourth fitting body 252 connected to the second third fitting body 251 according to an embodiment. The third fitting body 251 and the fourth fitting body 252 may be made of magnesium fluoride, D₂O, AlF₃, Fluental, CaF₂, Li₂CO₃, MgF₂, Al₂O₃, and Al. The third fitting body 251 and the fourth fitting body 252 may be made of a same material or different materials, and may be a cylinder, a square or other shapes, provided that the third fitting body and the fourth fitting body can match. When the cooling tube 240 is provided, the second fitting portion 250 may be provided with the second accommodating groove 253 capable of allowing the cooling tube 240 to pass through (refer to FIG. 16 below).

Hereinafter, MCNP software is used for simulation and calculation when the second fitting portion 250 is made of different materials in different embodiments. Table 2 below shows influences of beam quality factors in air in different embodiments (the unit of each noun in the table is the same as the above, and will not be repeated herein).

**Table 2 Beam quality in different embodiments**

| | The second fitting portion is not provided | The second fitting portion is made of MgF₂ | The second fitting portion is made of Al | The second fitting portion is made of CF₂ |
|---|---|---|---|---|
| Treatment depth | 10.62 | 10.73 | 10.74 | 10.66 |
| Maximal tumor dose | 60.67 | 66.69 | 63.87 | 64.46 |
| Epithermal neutron flux | 1.01E+09 | 9.51E+08 | 9.76E+08 | 9.65E+08 |
| Fast neutron contamination | 1.14E-12 | 8.56E-13 | 1.02E-12 | 8.80E-13 |

Compared with a case where the second fitting portion 250 is not provided, by providing the second fitting portion 250 between the particle transmission portion 210 and the second surface S2 of the accommodating cavity, the epithermal neutron flux is decreased slightly, but the fast neutron contamination is reduced significantly, and the maximal tumor dose and the treatment depth can be increased. This improves the quality of the neutron beam overall to achieve the better treatment effect.

Referring to FIG. 16, FIG. 16 is a schematic structural view of the second fitting portion 250 according to an embodiment. In an embodiment, the fourth fitting body 252 surrounds the third fitting body 251. The third fitting body 251 may be a cylinder, a cube, or other shapes. The fourth fitting body 252 may be the same as or different from the third fitting body 251 in shape. For example, in an embodiment, the third fitting body 251 is a cylinder. The fourth fitting body 252 is a tubular structure surrounding the third fitting body 251, and can serve to support the third fitting body, such that the third fitting body 251 surrounds the principal axis X of the neutron beam N, and the neutrons generated by the neutron generation portion 220 can pass through the third fitting body 251. When the third fitting body 251 is made of a material capable of improving the beam quality, the quality of the neutron beam N can be improved by the third fitting body 251. When the BSA 300 includes the moderator 310, the material of the third fitting body 251 may be the same as or different from the material of the moderator 310. The second accommodating groove 253 configured to accommodate the cooling tube 240 may further be formed in the second fitting portion 250.

The technical features of the foregoing embodiments can be employed in arbitrary combinations. For brevity of description, not all possible combinations of the technical features of the foregoing embodiments are described. However, the combinations of the technical features should be construed as falling within the scope described in this specification as long as there is no contradiction in the combinations.

Only several implementations of the present disclosure are described in detail in the foregoing embodiments, but they should not therefore be construed as limiting the scope of the present disclosure. It should be noted that those of ordinary skill in the art can further make variations and improvements without departing from the conception of the present disclosure. These variations and improvements all fall within the protection scope of the present disclosure. Therefore, the protection scope of this application shall be subject to the appended claims.

## Claims

1. A neutron capture therapy system, comprising:
a charged particle generation device configured to generate charged particles;
an accelerator configured to accelerate the charged particles;
a neutron generation device comprising a particle transmission portion and a neutron generation portion provided in a first end of the particle transmission portion, wherein the charged particles are capable of being interacted with the neutron generation portion through the particle transmission portion to generate neutrons;
a beam shaping assembly (BSA) provided with an accommodating cavity, wherein the accommodating cavity comprises a first surface and a second surface intersecting with the first surface, and the first end is provided in the accommodating cavity; and
a first fitting portion provided between the first surface and an outer surface of the particle transmission portion, wherein the first fitting portion surrounds at least a part of the particle transmission portion.

2. The neutron capture therapy system according to claim 1, wherein at least a part of the first fitting portion is attached to the outer surface of the particle transmission portion.

3. The neutron capture therapy system according to claim 1, wherein a first accommodating groove is formed in the first fitting portion; and at least a cooling tube or a cable is capable of passing through the first accommodating groove.

4. The neutron capture therapy system according to claim 1, wherein the particle transmission portion is a hollow cylinder; and at least a part of the first fitting portion surrounds a cylindrical outer surface of the particle transmission portion.

5. The neutron capture therapy system according to claim 1, wherein a material of the first fitting portion comprises one or more of a group consisting of lead, graphite, and Teflon.

6. The neutron capture therapy system according to claim 5, wherein the first fitting portion comprises at least a first fitting body and a second fitting body; the first fitting body and the second fitting body are made of a same material or different materials; and the first fitting body and the second fitting body are capable of being connected to each other.

7. The neutron capture therapy system according to claim 1, further comprising a second fitting portion provided between the particle transmission portion and the second surface of the accommodating cavity.

8. The neutron capture therapy system according to claim 7, wherein at least a part of the first fitting portion is attached to the second fitting portion.

9. The neutron capture therapy system according to claim 1, wherein the first fitting portion surrounds at least all of the particle transmission portion located in the accommodating cavity.

10. The neutron capture therapy system according to claim 1, wherein the first fitting portion is provided on the particle transmission portion through a fastener.

11. The neutron capture therapy system according to claim 8, wherein the second fitting portion is provided with a first end surface and a second end surface; the first end surface of the second fitting portion abuts against the second surface of the accommodating cavity; and at least a part of the second end surface of the second fitting portion abuts against the particle transmission portion.

12. The neutron capture therapy system according to claim 7, wherein the second fitting portion has a same shape and a same size as the second surface of the accommodating cavity, such that the second fitting portion is capable of completely filling the accommodating cavity, and is attached to the second surface of the accommodating cavity.

13. The neutron capture therapy system according to claim 7, wherein the second fitting portion comprises a third fitting body and a fourth fitting body connected to the third fitting body; and the fourth fitting body surrounds the third fitting body.

14. The neutron capture therapy system according to claim 13, wherein the BSA comprises a moderator configured to slow the neutrons generated by the neutron generation portion down to an energy range of epithermal neutrons; and the third fitting body and the moderator are made of a same material.

15. The neutron capture therapy system according to claim 7, wherein a material of the second fitting portion comprises one or more of a group consisting of magnesium fluoride, D₂O, AlF₃, Fluental, CaF₂, Li₂CO₃, MgF₂, Al₂O₃, and Al.
